(19) **Europäisches Patentamt / European Patent Office / Office européen des brevets**

(11) **EP 4 736 664 A1**

(12) # EUROPEAN PATENT APPLICATION
published in accordance with Art. 153(4) EPC

(43) Date of publication:
06.05.2026 Bulletin 2026/19

(21) Application number: 24832043.4

(22) Date of filing: 27.06.2024

(51) International Patent Classification (IPC):
*A23J 3/14* (2006.01)   *A23J 3/00* (2006.01)
*A23L 29/10* (2016.01)   *A23L 33/185* (2016.01)
*A23L 33/195* (2016.01)   *C07K 14/37* (2006.01)
*C07K 14/81* (2006.01)   *C07K 14/415* (2006.01)
*C12N 15/29* (2006.01)

(52) Cooperative Patent Classification (CPC):
A23J 3/00; A23J 3/14; A23L 29/10; A23L 33/185;
A23L 33/195; C07K 14/37; C07K 14/415;
C07K 14/81

(86) International application number:
PCT/JP2024/023348

(87) International publication number:
WO 2025/005184 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.06.2023 JP 2023104979

(71) Applicant: AJINOMOTO CO., INC.
Chuo-ku
Tokyo 104-8315 (JP)

(72) Inventors:
• YAMAZAKI, Shunsuke
Kawasaki-shi, Kanagawa 210-8681 (JP)
• TAGAMI, Uno
Kawasaki-shi, Kanagawa 210-8681 (JP)
• TAKAHASHI, Kazutoshi
Kawasaki-shi, Kanagawa 210-8681 (JP)
• HIRAO, Yoshinori
Kawasaki-shi, Kanagawa 210-8681 (JP)
• KITAZAWA, Daisuke
Kawasaki-shi, Kanagawa 210-8681 (JP)

(74) Representative: Strehl & Partner mbB
Maximilianstrasse 54
80538 München (DE)

Remarks:
The complete document including Reference
Table(s) and the Sequence Listing(s) can be
downloaded from the EPO website

(54) **COMPOSITION FOR SUBSTITUTING EGG WHITE**

(57) Provided is a technology for substituting egg white in a food product. Serpins derived from plants or fungi are utilized to substitute egg white in a food product.

EP 4 736 664 A1

**Description**

TECHNICAL FIELD

An aspect of the present invention relates to a technology for substituting egg white in a food product. Another aspect of the present invention relates to a technology for binding a food material. Another aspect of the present invention relates to a technology for emulsifying fats and oils in a food material.

BACKGROUND ART

Egg white has an ability to coagulate upon heating (thermal gelation ability) and is used as, for example, a binding agent for a food material.

Methylcellulose and polysaccharide thickeners are known as substitutes for egg white. However, no protein is known to have heat gelation ability equivalent to that of egg white.

Serpins are a class of proteins found as serine protease inhibitors (Non-patent document 1). Serpins are widely found, for example, throughout the plant kingdom. Approximately 50,000 structural homologues of serpins are registered in protein databases.

PRIOR ART DOCUMENTS

Non-patent documents

[Non-patent document 1] Nardy Lampl et al., Arabidopsis AtSerpin1, Crystal Structure and in Vivo Interaction with Its Target Protease RESPONSIVE TO DESICCATION-21 (RD21), J Biol Chem. 2010 Apr 30; 285(18): 13550-13560.
[Non-patent document 2] Niwa Tatsuya et al., Bimodal protein solubility distribution revealed by an aggregation analysis of the entire ensemble of Escherichia coli proteins, Proc Natl Acad Sci USA 106:4201-4206.

SUMMARY OF THE INVENTION

PROBLEMS TO BE ACHIEVED BY THE INVENTION

An aspect of the present invention is to provide a technology for substituting egg white in a food product. Another aspect of the present invention is to provide a technology for binding a food material. Another aspect of the present invention is to provide a technology for emulsifying fats and oils in a food material.

The inventors of the present invention conducted diligent studies in order to achieve the aforementioned objects. As a result, they found that by utilizing a serpin derived from a plant or fungus, egg white in a food product can be substituted, a food material can be bound, and/or fats and oils in a food material can be emulsified.

Accordingly, the present invention can be illustrated as follows.

[1] A composition comprising a serpin derived from a plant or fungus, for substituting egg white, for binding a food material, for emulsifying fats and oils in a food material, or for producing a food product.
[2] The composition mentioned above (specifically the composition according to [1]), wherein the serpin is a serpin derived from a plant of the family *Brassicaceae, Malvaceae, Euphorbiaceae, Asteraceae, Theaceae,* or *Apiaceae.*
[3] The composition mentioned above (specifically the composition according to [1] or [2]), wherein the serpin is a serpin derived from a plant of the genus *Arabidopsis, Brassica, Theobroma, Ricinus, Helianthus, Camellia,* or *Daucus.*
[4] The composition mentioned above (specifically the composition according to any of [1] to [3]), wherein the serpin is a serpin derived from *Arabidopsis thaliana, Brassica napus, Theobroma cacao, Ricinus communis, Helianthus annuus, Camellia sinensis* var. *sinensis,* or *Daucus carota* subsp. *sativus.*
[5] The composition mentioned above (specifically the composition according to any of [1] to [4]), wherein the serpin is the following protein (a), (b), or (c):

(a) a protein comprising the amino acid sequence of SEQ ID NO: 2, 4, 6, 8, 14, 16, or 18;
(b) a protein comprising an amino acid sequence of SEQ ID NO: 2, 4, 6, 8, 14, 16, or 18, but which includes substitution, deletion, insertion, and/or addition of 1 to 10 amino acid residues, wherein said protein has an egg white substitution function, a binding function, or an emulsifying function; or
(c) a protein comprising an amino acid sequence having an identity of 90% or higher to the amino acid sequence of SEQ ID NO: 2, 4, 6, 8, 14, 16, or 18, wherein said protein has an egg white substitution function, a binding function, or an emulsifying function.

[6] The composition mentioned above (specifically the composition according to any of [1] to [5]), wherein the serpin has a molecular weight of 40,000 to 50,000.

[7] The composition mentioned above (specifically the composition according to any of [1] to [6]), wherein the serpin has one or more properties selected from the following properties (1) to (4):

(1) the ratio of Ser residues in the amino acid sequence is 7.5% or higher;
(2) the isoelectric point (pI) is 4 to 7;
(3) the interior hydrophobicity is 1.5 or higher; and
(4) the predicted scaled solubility is 0.45 or higher.

[8] A method for producing a food product, the method comprising:
adding a serpin derived from a plant or fungus to a raw material of a food product.

[9] The method mentioned above (specifically the method according to [8]), wherein the food product to be produced is a food product in which egg white has been substituted, a food material has been bound, and/or fats and oils in a food material have been emulsified.

[10] A method for substituting egg white in a food product, the method comprising:
adding a serpin derived from a plant or fungus to a raw material of a food product.

[11] A method for binding a food material, the method comprising:
adding a serpin derived from a plant or fungus to a raw material of a food product.

[12] A method for emulsifying fats and oils in a food material, the method comprising:
adding a serpin derived from a plant or fungus to a raw material of a food product.

[13] The method mentioned above (specifically, the method according to any of [8] to [12]), wherein the serpin is a serpin derived from a plant of the family *Brassicaceae, Malvaceae, Euphorbiaceae, Asteraceae, Theaceae,* or *Apiaceae.*

[14] The method mentioned above (specifically, the method according to any of [8] to [13]), wherein the serpin is a serpin derived from a plant of the genus *Arabidopsis, Brassica, Theobroma, Ricinus, Helianthus, Camellia,* or *Daucus.*

[15] The method mentioned above (specifically, the method according to any of [8] to [14]), wherein the serpin is a serpin derived from *Arabidopsis thaliana, Brassica napus, Theobroma cacao, Ricinus communis, Helianthus annuus, Camellia sinensis* var. *sinensis,* or *Daucus carota* subsp. *sativus.*

[16] The method mentioned above (specifically, the method according to any of [8] to [15]), wherein the serpin is the following protein (a), (b), or (c):

(a) a protein comprising the amino acid sequence of SEQ ID NO: 2, 4, 6, 8, 14, 16, or 18;
(b) a protein comprising an amino acid sequence of SEQ ID NO: 2, 4, 6, 8, 14, 16, or 18, but which includes substitution, deletion, insertion, and/or addition of 1 to 10 amino acid residues, wherein said protein has an egg white substitution function, a binding function, or an emulsifying function; or
(c) a protein comprising an amino acid sequence having an identity of 90% or higher to the amino acid sequence of SEQ ID NO: 2, 4, 6, 8, 14, 16, or 18, wherein said protein has an egg white substitution function, a binding function, or an emulsifying function.

[17] The method mentioned above (specifically, the method according to any of [8] to [16]), wherein the serpin has a molecular weight of 40,000 to 50,000.

[18] The method mentioned above (specifically, the method according to any of [8] to [17]), wherein the serpin has one or more properties selected from the following properties (1) to (4):

(1) the ratio of Ser residues in the amino acid sequence is 7.5% or higher;
(2) the isoelectric point (pI) is 4 to 7;
(3) the interior hydrophobicity is 1.5 or higher; and
(4) the predicted scaled solubility is 0.45 or higher.

[19] The method mentioned above (specifically, the method according to any of [8] to [18]), wherein the serpin is added so that the concentration thereof at the time of eating is 0.05 to 30% (w/w).

[20] A food product comprising a serpin derived from a plant or fungus.

[21] The food product mentioned above (specifically the food product according to [20]), wherein the serpin is a serpin derived from a plant of the family *Brassicaceae, Malvaceae, Euphorbiaceae, Asteraceae, Theaceae,* or *Apiaceae.*

[22] The food product mentioned above (specifically the food product according to [20] or [21]), wherein the serpin is a serpin derived from a plant of the genus *Arabidopsis, Brassica, Theobroma, Ricinus, Helianthus, Camellia,* or *Daucus.*

[23] The food product mentioned above (specifically the food product according to any of [20] to [22]), wherein the serpin is a serpin derived from *Arabidopsis thaliana, Brassica napus, Theobroma cacao, Ricinus communis, Helianthus annuus, Camellia sinensis* var. *sinensis,* or *Daucus carota* subsp. *sativus.*

[24] The food product mentioned above (specifically the food product according to any of [20] to [23]), wherein the serpin is the following protein (a), (b), or (c):

(a) a protein comprising the amino acid sequence of SEQ ID NO: 2, 4, 6, 8, 14, 16, or 18;
(b) a protein comprising an amino acid sequence of SEQ ID NO: 2, 4, 6, 8, 14, 16, or 18, but which includes substitution, deletion, insertion, and/or addition of 1 to 10 amino acid residues, wherein said protein has an egg white substitution function, a binding function, or an emulsifying function; or
(c) a protein comprising an amino acid sequence having an identity of 90% or higher to the amino acid sequence of SEQ ID NO: 2, 4, 6, 8, 14, 16, or 18, wherein said protein has an egg white substitution function, a binding function, or an emulsifying function.

[25] The food product mentioned above (specifically the food product according to any of [20] to [24]), wherein the serpin has a molecular weight of 40,000 to 50,000.

[26] The food product mentioned above (specifically the food product according to any of [20] to [25]), wherein the serpin has one or more properties selected from the following properties (1) to (4):

(1) the ratio of Ser residues in the amino acid sequence is 7.5% or higher;
(2) the isoelectric point (pI) is 4 to 7;
(3) the interior hydrophobicity is 1.5 or higher; and
(4) the predicted scaled solubility is 0.45 or higher.

[27] The food product mentioned above (specifically the food product according to any of [20] to [26]), wherein the food product has a serpin content of 0.05 to 30% (w/w).

EFFECTS OF THE INVENTION

According to one aspect of the present invention, egg white in a food product can be substituted. According to one aspect of the present invention, a food material can be bound. According to one aspect of the present invention, fats and oils in a food material can be emulsified.

MODE FOR CARRYING OUT THE INVENTION

Hereafter, the present invention will be explained in detail.

<1> Active ingredient

In the present invention, a serpin is used as an active ingredient. A serpin is also referred to as the "active ingredient".

In one embodiment, by utilizing the active ingredient, egg white in a food product can be substituted, i.e., an effect of substituting egg white in a food product can be obtained. This effect is also referred to as "egg white substitution effect". In other words, in one embodiment, the active ingredient has a function of substituting egg white in a food product. This function is also referred to as "egg white substitution function". The substitution of egg white in a food product is also simply referred to as "substitution of egg white". The substitution of egg white may be substitution of a part of or whole egg white in a food product. In other words, "substitution of egg white" may mean reduction of egg white content in a food product. The "substitution of egg white" may mean, in other words, reduction in the amount of egg white used in production of a food product. By utilizing the active ingredient, content of egg white in a food product may be reduced to, for example, 0.9 times or lower, 0.8 times or lower, 0.7 times or lower, 0.6 times or lower, 0.5 times or lower, 0.4 times or lower, 0.3 times or lower, 0.2 times or lower, or 0.1 times or lower of the normal content of egg white in the food product, or may be reduced to zero. By utilizing the active ingredient, the amount of egg white used in production of a food product may be reduced to 0.9 times or lower, 0.8 times or lower, 0.7 times or lower, 0.6 times or lower, 0.5 times or lower, 0.4 times or lower, 0.3 times or lower, 0.2 times or lower, or 0.1 times or lower of the normal amount of egg white used in the production of the food product, or may be reduced to zero. By utilizing the active ingredient, specifically, the function of egg white may be substituted. That is, for example, the function of egg white may be substituted by the function of the active ingredient, thereby substituting egg white in a food product. Examples of the function of egg white include binding, emulsifying, foaming, and water-retaining functions. By utilizing the active ingredient, for example, one or more of these functions of egg white may be substituted. For example, one, two, three, or all four of these functions of egg white may be substituted by utilizing the active ingredient. Egg white includes egg white of eggs of birds such as chicken eggs. The egg white substitution function (e.g., binding function) can be measured, for example, as gelation ability during heating (also referred to as thermal gelation ability) of serpin. The thermal gelation ability can be measured by, for example, measuring storage modulus of serpin when it is heated and gelled. The storage modulus can be measured by, for example, using a rheometer. The thermal gelation ability can be specifically measured by, for example, the procedure described in the section of Examples.

In one embodiment, by utilizing the active ingredient, a food material can be bound, i.e., an effect of binding a food material can be obtained. This effect is also referred to as "binding effect". In other words, in one embodiment, the active ingredient has a function of binding a food material. This function is also referred to as the "binding function". Binding of food material is also referred to simply as "binding". Examples of food materials to be bound include protein-containing food materials such as meat and veggie meat. The active ingredient may function as, for example, a binder for a food material on the basis of the binding function thereof. The binding effect can be measured by, for example, measuring texture of a food product. Texture of a food product can be measured by, for example, sensory evaluation performed by a panel of experts. Texture of a food product can be measured, for example, by using a texture measuring device such as a texture analyzer. The binding function can also be measured, for example, as gelation ability during heating (also referred to as thermal gelation ability) of serpin. The thermal gelation ability can be measured by, for example, measuring storage modulus of serpin when it is heated and gelled. The storage modulus can be measured by, for example, using a rheometer. The thermal gelation ability can specifically be measured by, for example, the procedure described in the section of Examples.

In one embodiment, by utilizing the active ingredient, fats and oils in a food material can be emulsified, i.e., an effect of emulsifying fats and oils in a food material can be obtained. This effect is also referred to as "emulsifying effect". In other words, in one embodiment, the active ingredient has a function of emulsifying fats and oils in a food material. This function is also referred to as "emulsifying function". Emulsification of fats and oils in a food material is also referred to simply as "emulsification". Examples of food products containing fats and oils to be emulsified include beverages, ice confectioneries, confectioneries, breads, soups, liquid seasonings, processed meat products, processed seafood products, dairy products, health foods, medical foods, jams, preserves, syrups, fruits for processing, pickles, grain flours, noodles, liquid diets, and so forth, preferably food products other than health foods and medical foods. The active ingredient may function as, for example, an emulsifier on the basis of the emulsifying function thereof. The emulsifying effect can be evaluated by, for example, calculating creaming index (CI) of an emulsion. The creaming index (CI) is a numerical value representing ability to maintain oil droplets (white layer) generated by emulsification. Specifically, the creaming index (CI) can be calculated, for example, from the height of the entire emulsion (HE) and the height of the clear layer (HS) in accordance with the following equation.

$$\text{Creaming Index (CI)} = \text{HS/HE}$$

The term "serpin" means a protein that belongs to the serpin superfamily. Serpin may or may not function as, for example, a serine protease inhibitor. One type of serpin may be used, or a combination of two or more types of serpins may be used.

The serpin used as the active ingredient is a serpin derived from a plant or fungus. A serpin derived from organism X (X is a name identifying the organism) is also referred to as "an organism X-derived serpin". That is, for example, "plant-derived serpin" means serpin derived from a plant. Further, for example, "fungus-derived serpin" means serpin derived from a fungus. Specific examples of serpins are plant-derived serpins. The type of the plant or fungus from which serpin is derived is not particularly limited. Examples of such plants include plants of the families *Brassicaceae, Malvaceae, Euphorbiaceae, Asteraceae, Theaceae,* and *Apiaceae.* Examples of plants of the family *Brassicaceae* include plants of the genera *Arabidopsis, Brassica,* and *Raphanus.* Examples of the plants of the genus *Arabidopsis* include *Arabidopsis thaliana.* Examples of the plants of the genus *Brassica* include *Brassica napus* and *Brassica rapa* subsp. *pekinensis* (Chinese cabbage). Examples of the plants of the genus *Raphanus* include *Raphanus sativus* (radish). Examples of the plants of the family *Malvaceae* include plants of the genus *Theobroma.* Examples of the plants of the genus *Theobroma* include *Theobroma cacao* (cacao). Examples of the plants of the family *Euphorbiaceae* include plants of the genus *Ricinus.* Examples of the plants of the genus *Ricinus* include *Ricinus communis* (castor bean). Examples of the plants of the family *Asteraceae* include plants of the genus *Helianthus.* Examples of the plants of the genus *Helianthus* include *Helianthus annuus* (sunflower). Examples of the plants of the family *Theaceae* include plants of the genus *Camellia.* Examples of the plants of the genus *Camellia* include *Camellia sinensis* var. *sinensis* (tea plant). Examples of the plants of the family *Apiaceae* include plants of the genus *Daucus.* Examples of the plants of the genus *Daucus* include *Daucus carota* subsp. *sativus* (carrot). Specific examples of the plants include *Arabidopsis thaliana, Brassica napus, Theobroma cacao,* and *Ricinus communis.* A gene encoding serpin is also referred to as "serpin gene". Nucleotide sequences of serpin genes derived from various organisms and amino acid sequences of serpins encoded by them can be obtained from, for example, public databases such as NCBI and technical literature such as patent documents. The nucleotide sequence of the serpin gene of *Arabidopsis thaliana* and the amino acid sequence of the serpin encoded by the gene are shown as sequences of SEQ ID NOS: 1 and 2, respectively. The nucleotide sequence of the serpin gene of *Brassica napus* and the amino acid sequence of the serpin encoded by the gene are shown as sequences of SEQ ID NOS: 3 and 4, respectively. The nucleotide sequence of the serpin gene of *Theobroma cacao* and the amino acid sequence of the serpin encoded by the gene are shown as sequences of SEQ ID NOS: 5 and 6, respectively. The nucleotide sequence of the serpin gene of *Ricinus communis* and the amino acid sequence of the serpin encoded by the gene are shown as sequences of SEQ ID NOS: 7 and 8, respectively. The nucleotide sequence of the serpin gene of *Brassica rapa* subsp. *pekinensis* and the amino acid sequence of the serpin encoded by the gene are shown as sequences of SEQ ID NOS: 9 and 10, respectively. The nucleotide sequence of the serpin gene of *Raphanus sativus* and the amino acid sequence of the serpin encoded by the gene are shown as sequences of SEQ ID NOS: 11 and 12, respectively. The nucleotide sequence of the serpin gene of *Helianthus annuus* and the amino acid sequence of the serpin encoded by the gene are shown as sequences of SEQ ID NOS: 13 and 14, respectively. The nucleotide sequence of the serpin gene of *Camellia sinensis* var. *sinensis* and the amino acid sequence of the serpin encoded by the gene are shown as sequences of SEQ ID NOS: 15 and 16, respectively. The nucleotide sequence of the serpin gene of *Daucus carota* subsp. *sativus* and the amino acid sequence of the serpin encoded by the gene are shown as sequences of SEQ ID NOS: 17 and 18, respectively. That is, the serpin gene may be, for example, a gene having any of the nucleotide sequences of the serpin genes exemplified above (e.g., the nucleotide sequences of SEQ ID NOS: 1, 3, 5, 7, 9, 11, 13, 15, and 17). The serpin may be, for example, a protein having any of the amino acid sequences of serpins exemplified above (e.g., the amino acid sequences of SEQ ID NOS: 2, 4, 6, 8, 10, 12, 14, 16, and 18). The expression "having an (amino acid or nucleotide) sequence" means "comprising the (amino acid or nucleotide) sequence" and includes "consisting of the (amino acid or nucleotide) sequence", unless especially noted.

The serpin gene may be a variant of the serpin genes exemplified above (e.g., genes having the nucleotide sequences of SEQ ID NOS: 1, 3, 5, 7, 9, 11, 13, 15, and 17) as long as the original function thereof is maintained. Similarly, the serpin may be a variant of the serpins exemplified above (e.g., proteins having the amino acid sequences of SEQ ID NOS: 2, 4, 6, 8, 10, 12, 14, 16, and 18) as long as the original function thereof is maintained. Such a variant that maintains the original function may be referred to as "a conservative variant". The term "serpin gene" shall encompass the serpin genes exemplified above as well as conservative variants thereof. Similarly, the term "serpin" shall encompass the serpins exemplified above as well as conservative variants thereof. Further, both serpin gene and serpin specified by a species of organism from which they are derived are not limited to those actually possessed by the species of organism, and may encompass conservative variants thereof (which may or may not be actually possessed by the species), unless especially noted. Examples of such conservative variants include, for example, homologues as well as artificial modifications of the serpin genes and serpins exemplified above. The term "serpin derived from organism X" is not limited to serpin found in the organism X, but may also be an artificial modification of serpin found in the organism X. Such serpin derived from the organism X may be, for example, one obtained by producing it in the organism X or one obtained by heterologous expression (i.e., recombinant protein). For example, *Arabidopsis thaliana-derived* serpin may be, for example, one obtained by producing it in *Arabidopsis thaliana* cells, or may be one obtained by heterologous expression (i.e., recombinant protein).

The expression that "original function is maintained" means that a variant of gene or protein has a function (e.g., activity or property) that corresponds to the function (e.g., activity or property) of the original gene or protein. That is, the expression that "original function is maintained" used for a gene may mean that a variant of the gene encodes a protein that maintains the original function. That is, the expression that "original function is maintained" used for a serpin gene may mean that a variant of the gene encodes serpin. The expression that "original function is maintained" used for serpin may mean that a variant of a protein has the desired function as serpin (e.g., egg white substitution function, binding function, or emulsifying function) and the level of these functions relative to the original protein is maintained at 10% or more, 30% or more, 50% or more, or 80% or more.

Hereafter, examples of conservative variants will be explained.

Homologues of serpin gene or homologues of serpin can be easily obtained, for example, from public databases by BLAST or FASTA searches using any of the nucleotide sequences of the serpin genes exemplified above or amino acid sequences of serpins exemplified above as query sequences. Homologues of serpin gene can also be obtained by, for example, PCR using chromosomes of various organisms as a template and oligonucleotides prepared on the basis of these known serpin gene sequences as primers.

The serpin gene may be a gene encoding a protein having any of the above amino acid sequences (for example, the amino acid sequences of SEQ ID NOS: 2, 4, 6, 8, 10, 12, 14, 16, and 18), but which includes substitution, deletion, insertion, and/or addition of one or several amino acids at one or several positions, as long as the original function thereof is maintained. The encoded protein may have, for example, an extended or shortened N- and/or C-terminus. The term "one or several" mentioned above specifically means, although it depends on positions and types of amino acid residues in the three-dimensional structure of the protein, for example, 1 to 50 residues, 1 to 40 residues, or 1 to 30 residues, preferably 1 to 20 residues, more preferably 1 to 10 residues, further preferably 1 to 5 residues, especially preferably 1 to 3 residues.

The above substitution, deletion, insertion, and/or addition of one or several amino acids is a conservative mutation that maintains function of the protein in a normal state. A typical conservative mutation is a conservative substitution. The conservative substitution is a mutation caused by substitution occurring among Phe, Trp, and Tyr when the amino acid to be substituted is an aromatic amino acid, among Leu, Ile, and Val when it is a hydrophobic amino acid, between Gln and Asn when it is a polar amino acid, among Lys, Arg, and His when it is a basic amino acid, between Asp and Glu when it is an acidic amino acid, and between Ser and Thr when it is an amino acid with hydroxyl group. Specific examples of substitutions that are considered as conservative substitutions include substitutions of Ala with Ser or Thr; Arg with Gln, His or Lys; Asn with Glu, Gln, Lys, His or Asp; Asp with Asn, Glu or Gln; Cys with Ser or Ala; Gln with Asn, Glu, Lys, His, Asp or Arg; Glu with Gly, Asn, Gln, Lys or Asp; Gly with Pro; His with Asn, Lys, Gln, Arg or Tyr; Ile with Leu, Met, Val or Phe; Leu with Ile, Met, Val or Phe; Lys with Asn, Glu, Gln, His or Arg; Met with Ile, Leu, Val or Phe; Phe with Trp, Tyr, Met, Ile or Leu; Ser with Thr or Ala; Thr with Ser or Ala; Trp with Phe or Tyr; Tyr with His, Phe or Trp, and Val with Met, Ile or Leu. The substitution, deletion, insertion, or addition of amino acids mentioned above also include those caused by naturally occurring mutations due to individual differences or differences of species of the organism from which the gene is derived (mutants or variants).

The serpin gene may be a gene encoding a protein having an amino acid sequence having an identity of, for example, 55% or higher, 60% or higher, 65% or higher, 70% or higher, 75% or higher, 80% or higher, or 85% or higher, preferably 90% or higher, more preferably 95% or higher, even more preferably 97% or higher, especially preferably 99% or higher, to the total amino acid sequence of any of the aforementioned amino acid sequences, as long as the original function thereof is maintained.

The serpin gene may also be a gene (e.g., DNA) that hybridizes under stringent conditions with a probe, such as a sequence complementary to the whole or part of any of the sequences mentioned above, which can be prepared from any of the nucleotide sequences mentioned above (e.g., the sequence shown as SEQ ID NO: 1, 3, 5, 7, 9, 11, 13, 15, or 17), so long as the original function thereof is maintained. The term "stringent conditions" refers to conditions under which so-called specific hybrids are formed and nonspecific hybrids are not formed. To give examples, there are mentioned conditions under which DNAs showing a high identity, such as 55% or higher, 60% or higher, 65% or higher, 70% or higher, 75% or higher, 80% or higher, or 85% or higher, preferably 90% or higher, more preferably 95% or higher, even more preferably 97% or higher, especially preferably 99% or higher to each other hybridize with each other, and DNAs showing an identity lower than the above levels do not hybridize with each other, and the washing conditions of the usual Southern hybridization, i.e., one time, preferably 2 or 3 times, of washing at a salt concentration and temperature of 60°C, 1xSSC, and 0.1% SDS, preferably 60°C, 0.1xSSC, and 0.1% SDS, more preferably 68°C, 0.1xSSC, and 0.1% SDS.

As described above, the probe used for the hybridization mentioned above may be a part of a complementary sequence of the gene. Such a probe can be prepared by PCR using oligonucleotides prepared on the basis of a known gene sequence as primers and a DNA fragment containing the gene described above as a template. For example, a DNA fragment of about 300 bp in length can be used as such a probe. When a DNA fragment of about 300 bp in length is used as a probe, the washing conditions for hybridization consist of, for example, 50°C, 2xSSC, and 0.1% SDS.

Since codon degeneracy is different from host to host, the serpin gene may have any codon substituted with an equivalent codon. That is, the serpin gene may be a variant of any of the serpin genes exemplified above caused by degeneracy of genetic code. For example, an expression-suppressing gene may be modified to have codons optimal for the frequency of codon usage in the host used.

The "identity" between amino acid sequences means the identity between amino acid sequences calculated by using blastp with the default settings of Scoring Parameters (Matrix, BLOSUM62; Gap Costs, Existence=11, Extension=1; Compositional Adjustments, Conditional compositional score matrix adjustment). The "identity" between nucleotide sequences means the identity between nucleotide sequences calculated by using blastn with the default settings of Scoring Parameters (Match/Mismatch Scores=1, -2; Gap Costs=Linear).

As the serpin, for example, a commercially available product may be used, or one obtained by appropriately producing it may be used.

The method for producing serpin is not particularly limited. For example, serpin can be produced by culturing host cells that produce serpin. The host cells are not particularly limited as long as they can express serpin. Examples of such host cells include cells used for heterologous expression of proteins. Specific examples of the host cells include bacteria, fungi, plant cells, animal cells, and insect cells. Specific examples of the host cells include, in particular, microorganisms such as bacteria. Examples of the bacteria include bacteria of the genus *Escherichia* such as *Escherichia coli* and the bacteria of the genus *Corynebacterium* such as *Corynebacterium glutamicum.* Serpin-producing host cells may be those that inherently produce serpin or may be those modified to produce serpin. Host cells that produce serpin can be obtained by, for example, introducing a gene encoding serpin into the host cells so as to allow expression. The culture conditions of the serpin-producing host cells are not particularly limited as long as serpin is produced. For example, host cells that produce serpin can be cultured under the usual conditions for culturing host cells.

Serpin may or may not contain any ingredient other than serpin. Serpin may contain, for example, another enzyme. That is, as the serpin, purified serpin may be used, or a material containing serpin may be used. Examples of such materials containing serpin include cultures of host cells that produce serpin, culture supernatants separated from such cultures, host cells separated from such cultures, and processed products of these. Examples of the processed products include cell lysate of host cells. When serpin forms inclusion bodies in the host cells, serpin can be solubilized as appropriate. Serpin may be purified to a desired degree.

If a material containing serpin is used, the amount of serpin (e.g., content (concentration) or amount used) shall be calculated on the basis of the amount of serpin itself in the material.

Serpin may have a molecular weight of, for example, 40,000 to 50,000. Serpins of *Arabidopsis thaliana, Brassica napus, Theobroma cacao* (cacao), *Ricinus communis* (castor bean), *Brassica rapa* subsp. *pekinensis* (Chinese cabbage), *Raphanus sativus* (radish), *Helianthus annuus* (sunflower), *Camellia sinensis* var. *sinensis* (tea plant), and *Daucus carota* subsp. *sativus* (carrot) can all have a molecular weight of 40,000 to 50,000.

Serpin may have, for example, one or more properties selected from those described in the following (1) to (4). Serpin may have, for example, one, two, three, or all four of the properties selected from those described in the following (1) to (4). Serpins of *Arabidopsis thaliana, Brassica napus, Theobroma cacao* (cacao), *Ricinus communis* (castor bean), *Brassica rapa* subsp. *pekinensis* (Chinese cabbage), *Raphanus sativus* (radish), *Helianthus annuus* (sunflower), *Camellia sinensis* var. *sinensis* (tea plant), and *Daucus carota* subsp. *sativus* (carrot) may have all of the following properties (1) to (4).

(1) The ratio of Ser residues in the amino acid sequence is 7.5% or higher.
(2) The isoelectric point (pI) is 4 to 7.
(3) The interior hydrophobicity is 1.5 or higher.
(4) The predicted scaled solubility is 0.45 or higher.

For the property (3) mentioned above, the term "interior hydrophobicity" means the interior hydrophobicity calculated from data of the AlphaFold Protein Structure Database (AlphaFold 2 DB; https://alphafold.ebi.ac.uk) by using Biopython version 1.79 (https://biopython.org) according to the following procedure. That is, SASA values of amino acid residues constituting serpin are calculated by using Biopython, and amino acid residues having a SASA value of less than 20 are considered to be "interior" amino acid residues. Then, the total of the values on the Kyte-Doolittle scale (i.e., hydrophobicity indexes of amino acids described in Kyte, J. and Doolittle, R. F. (1982). A simple method for displaying the hydropathic character of a protein. J Mol Biol, 157(1):105-132, Table 2) of all the "interior" amino acid residues is calculated as the "interior hydrophobicity".

As for the property (4) mentioned above, the term "predicted scaled solubility" is a value for predicting solubility of a protein, calculated by using the Protein-Sol software (https://www.manchester.ac.uk) with the default parameters. Proteins with a predicted scaled solubility of 0.45 or higher are predicted to have higher solubility than the average soluble proteins of *E. coli.* The term "average soluble proteins of *E. coli"* mentioned here refers to the proteins of the experimental solubility dataset defined in the experiment of Non-patent document 2 (Niwa et al 2009).

<2> Composition of the present invention

The composition of the present invention is a composition comprising the active ingredient (i.e., serpin).

In one aspect, by using the composition of the present invention, egg white in a food product can be substituted, i.e., the egg white substitution effect can be obtained. In other words, the composition of the present invention has an egg white substitution function. Therefore, the composition of the present invention may be used to substitute egg white. That is, the composition of the present invention may be, for example, a composition for substituting egg white.

In one aspect, by using the composition of the present invention, a food material can be bound, i.e., a binding effect can be obtained. In other words, the composition of the present invention has a binding function. Therefore, the composition of the present invention may be used for binding a food material. That is, the composition of the present invention may be, for example, a composition for binding a food material.

In one aspect, by using the composition of the present invention, fats and oils in a food material can be emulsified, i.e., an emulsifying effect can be obtained. In other words, the composition of the present invention has an emulsifying function. Therefore, the composition of the present invention may be used for emulsifying fats and oils in a food material. That is, the composition of the present invention may be, for example, a composition for emulsifying fats and oils in a food material.

Further, by using the composition of the present invention, food products in which egg white has been substituted, a food material has been bound, and/or fats and oils in a food material have been emulsified can be produced. Therefore, the composition of the present invention may be used in production of a food product (specifically, production of a food product in which egg white has been substituted, a food material has been bound, and/or fats and oils in a food material have been emulsified). That is, the composition of the present invention may be, for example, a composition for producing a food product (specifically, production of a food product in which egg white has been substituted, a food material has been bound, and/or fats and oils in a food material have been emulsified).

The composition of the present invention may be, for example, a seasoning. Specifically, the composition of the present invention may be, for example, a seasoning for substituting egg white, a seasoning for binding a food material, a seasoning for emulsifying fats and oils in a food material, or a seasoning for producing a food product (specifically, production of a food product in which egg white has been substituted, a food material has been bound, and/or fats and oils in a food material have been emulsified).

The composition of the present invention may be utilized for substituting egg white, binding a food material, emulsifying fats and oils in a food material, or producing food products in the manners described in the Method section below.

The composition of the present invention may consist of the active ingredient or may contain ingredients other than the active ingredient. That is, the active ingredient may be used as it is or in combination with other ingredients as the composition of the present invention.

The ingredients other than the active ingredient are not particularly limited as long as the desired effect (e.g., egg white substitution effect, binding effect or emulsifying effect) is not lost (i.e., the desired effect exerted by the active ingredient is obtained). The ingredients other than the active ingredient can be selected as appropriate according to various conditions, such as type of food product. Examples of the ingredients other than the active ingredient include ingredients used in food products or pharmaceuticals. Specific examples of the ingredients other than the active ingredient include, for example, the raw materials of food products, which will be described later.

The composition of the present invention may be made, for example, in the form of a preparation as appropriate. In the production of such a preparation, additives may be used as appropriate. Examples of the additives include excipients, binders, disintegrants, lubricants, stabilizers, flavoring agents, diluents, surfactants, and solvents. The additives can be appropriately selected according to various conditions such as, for example, the form of the composition of the present invention.

The form of the composition of the present invention is not particularly limited. The composition of the present invention may be in any form, for example, powder, flakes, tablets, paste, liquid, etc.

The contents and content ratios of the ingredients in the composition of the present invention (i.e., active ingredient and optionally other ingredients) are not particularly limited as long as the desired effect (e.g., egg white substitution effect, binding effect or emulsifying effect) is obtained. The contents and content ratios of the ingredients in the composition of the present invention can be appropriately set according to various conditions, such as the intended use of the composition of the present invention.

The content of the active ingredient in the composition of the present invention is higher than 0% (w/w) and 100% (w/w) or lower. The content of the active ingredient in the composition of the present invention may be, for example, 0.1% (w/w) or higher, 0.2% (w/w) or higher, 0.5% (w/w) or higher, 1% (w/w) or higher, 2% (w/w) or higher, 5% (w/w) or higher, 10% (w/w) or higher, 20% (w/w) or higher, 30% (w/w) or higher, 50% (w/w) or higher, or 70% (w/w) or higher, or may be 100% (w/w) or lower, 99.9% (w/w) or lower, 90% (w/w) or lower, 70% (w/w) or lower, 50% (w/w) or lower, 30% (w/w) or lower, 20% (w/w) or lower, 10% (w/w) or lower, 5% (w/w) or lower, 1% (w/w) or lower, 0.5% (w/w) or lower, or 0.2% (w/w) or lower, or may be any combination that does not contradict with the above-described ranges. The content of the active ingredient in the composition of the invention may specifically be, for example, 0.1 to 1% (w/w), 1 to 10% (w/w), 10 to 30% (w/w), 30 to 50% (w/w), 50 to 70% (w/w), 70 to 90% (w/w), or 70 to 100% (w/w). The content of the active ingredient in the composition of the invention may specifically be, for example, 1 to 100% (w/w), 5 to 90% (w/w), or 10 to 70% (w/w). When the composition of the present invention contains two or more types of serpins, the contents of those two or more types of serpins in the composition of the present invention may be independently set or the total thereof may be set to be within any of the ranges of the content of the active ingredient in the composition of the present invention exemplified above (provided that the total content of such two or more kinds of serpins in the composition of the present invention is 100% (w/w) or lower). When the composition of the present invention contains two or more kinds of serpins, the "content of the active ingredient in the composition of the present invention" shall mean the total content of those two or more types of serpins in the composition of the present invention, unless especially noted.

The contents of the respective ingredients in the composition of the present invention (i.e., the active ingredient and optionally other ingredients) can be set so that, for example, the amounts of the ingredients added in the method of the present invention described below can be obtained.

The respective ingredients contained in the composition of the present invention (i.e., the active ingredient and optionally other ingredients) may be contained in the composition of the present invention as a mixture of them, separately from each other, or separately in any combination thereof. For example, the composition of the present invention may be provided as a set of the respective ingredients, each packaged separately. In such a case, the ingredients in the set may be used together as appropriate at the time of use.

<3> Method of the present invention

The method of the present invention is a method comprising the step of utilizing the active ingredient (i.e., serpin).

In one aspect, egg white in a food product can be substituted by the method of the present invention, specifically by utilizing the active ingredient. That is, the egg white substitution effect can be obtained. Therefore, the method of the present invention may be implemented for substituting egg white in a food product. That is, the method of the present invention may be, for example, a method for substituting egg white in a food product. This method is also referred to as the "method for substituting egg white of the present invention".

In one aspect, a food material can be bound by the method of the present invention, specifically by utilizing the active ingredient. That is, the binding effect can be obtained. Therefore, the method of the present invention may be implemented for binding a food material. That is, the method of the present invention may be, for example, a method for binding a food material. This method is also referred to as the "binding method of the present invention".

In one aspect, fats and oils in a food material can be emulsified by the method of the present invention, specifically, by utilizing the active ingredient. That is, the emulsifying effect can be obtained. Therefore, the method of the present invention may be implemented for emulsifying fats and oils in a food material. That is, the method of the present invention may be, for example, a method for emulsifying fats and oils in a food material. This method is also referred to as the "emulsification method of the present invention".

Further, by the method of the present invention, specifically by utilizing the active ingredient, food products in which egg white has been substituted, a food material has been bound, and/or fats and oils in a food material have been emulsified can also be produced. Therefore, the method of the present invention may be implemented for producing a food product (specifically, producing a food product in which egg white has been substituted, a food material has been bound, and/or fats and oils in a food material have been emulsified). That is, the method of the present invention may be, for example, a method for producing a food product (specifically, producing a food product in which egg white has been substituted, a food material has been bound, and/or fats and oils in a food material have been emulsified). This method is also referred to as the "method for producing a food product of the present invention".

The active ingredient can be used to substitute egg white, bind a food material, emulsify fats and oils in a food material, or produce a food product by adding the active ingredient to a raw material of a food product during the production of the food product. That is, the use of the active ingredient includes adding the active ingredient to a raw material of a food product. That is, the method of the present invention may specifically be a method for substituting egg white in a food product, comprising, for example, adding the active ingredient to a raw material of a food product. The method of the present invention may also specifically be a method for binding a food material, comprising, for example, adding the active ingredient to a raw material of a food product. The method of the present invention may also specifically be a method for emulsifying fats and oils in a food material, comprising, for example, adding the active ingredient to a raw material of a food product. The method of the present invention may also specifically be a method for producing a food product (specifically, producing a food product in which egg white has been substituted, a food material has been bound, and/or fats and oils in a food material have been emulsified), comprising, for example, adding the active ingredient to a raw material of a food product. The term "blend" is also used to mean "add".

The active ingredient may be utilized in the method of the present invention, for example, in the form of the composition of the present invention. That is, "use of the active ingredient" includes use of the composition of the present invention. For example, "addition of the active ingredient" includes addition of the composition of the present invention.

A food product obtained by the method of the present invention is also referred to as "food product of the present invention". The food product of the present invention is specifically a food product in which egg white has been substituted, a food material has been bound, and/or fats and oils in a food material have been emulsified. The food product of the present invention is, in other words, a food product to which the active ingredient has been added. The food product of the present invention is also, in other words, a food product comprising the active ingredient.

Substitution of egg white, binding of a food material, emulsification of a food material, or production of a food product may be carried out, for example, in the same manner as in the production of ordinary food products, except for the use of the active ingredient. That is, the substitution of egg white, binding of a food material, emulsification of a food material, or production of a food product may be carried out, for example, by using the same raw materials under the same production conditions as those for ordinary food products, except that the active ingredient is utilized. In addition, any of such raw materials and production conditions of food products may be used for substitution of egg white, binding of a food material, emulsification of fats and oils in a food material, or production of a food product with appropriate modification.

The food product is not particularly limited as long as it is a food product for which the desired effect such as egg white substitution effect, binding effect, or emulsifying effect is desired to be achieved. Examples of the food products include those containing egg white. Examples of the food products also include food products that do not contain egg white. The food product may be a food product that can typically contain egg white, but of which egg white content is reduced. The food product may also be a beverage or a seasoning. The food product may be, for example, liquid or solid.

Specific examples of the food products (e.g., food products that can typically contain egg white) include egg-containing sauces such as carbonara sauce and custard cream, food products using such sauces, egg-bound foods such as oyakodon (rice bowl dish topped with chicken and other ingredients bound with simmered egg), katsudon (rice bowl dish topped with pork cutlet and other ingredient bound with simmered egg) and tenshinhan (rice dish topped with crab omelet with starchy sauce), baked egg foods such as dashimaki-tamago (Japanese rolled omelet made with egg and dashi (Japanese stock)), foods containing fried egg such as niratama (fried leek and egg) and Chinese fried rice, confectionaries such as marshmallows, macaroons, cakes, and cookies, chawanmushi (dashi-taste steamed egg custard), egg tofu, omurice (omelet stuffed with fried rice), egg sandwiches, and pudding. Examples of food products using carbonara sauce include carbonara spaghetti (pasta). Examples of food products using custard cream include cream puffs.

Specific examples of the food products also include those produced by processing a protein-containing food material. Examples of the protein-containing food materials include meat and veggie meat. The term "meat" means edible meat. Examples of the meat include animal meat. The term "animal" may mean any organism classified in the animal kingdom (*Animalia*). Examples of the animals include vertebrates and aquatic organisms. Examples of the vertebrates include mammals, birds, reptiles, amphibians, and fish. Examples of the vertebrates include, in particular, mammals, birds, and fish. Examples of the mammals include domestic animals such as cattle, pigs, horses, sheep, goats, and rabbits; wild animals such as boars, deer, and bears; and marine mammals such as whales, dolphins, and sea lions. Examples of the birds include chickens, turkeys, ducks, geese, guinea fowls, quails, and ostriches. Examples of the fish include horse mackerel, salmon, cod, puffer fish, Japanese whiting, conger eel, hoki (blue grenadier), hake, eel, tuna, longtooth grouper, and sea bream. Examples of the aquatic organisms include, in addition to aquatic mammals and fish, crustaceans such as shrimps and crabs, shellfish such as scallops and oysters, and other seafood such as squids and octopuses. The term "veggie meat" may mean a food product produced by processing a plant protein-containing raw material into a meat-like product or a food product produced from such a product. Examples of plants from which plant proteins are derived include beans and grains such as wheat. Examples of beans include soybeans, peas, broad beans, chickpeas, almonds, peanuts, and lupin beans. Examples of grains include wheat, barley, and rye. Examples of the plant proteins include, in particular, bean proteins. Examples of the plant proteins include, more specifically, soy proteins. That is, examples of the veggie meat include, in particular, those produced from a soy protein-containing raw material (also referred to as "soy meat"). Examples of food products produced by processing protein-containing food materials include hams, sausages, hamburger steaks, meat dumplings, and fish paste products. Meat dumplings may include, for example, those used as filling of noodle dough-wrapped filling type foods such as shaomai, and those used as ingredients in cabbage rolls. Examples of sausages and hamburger steaks include, for example, sausages and hamburgers produced by processing veggie meat such as soybean meat, and sausages and hamburger steaks produced by processing meat such as beef, pork, and fish meat. Fish paste products include kamaboko, crab kamaboko, and fried kamaboko. In these food products, the active ingredient may function, for example, as a binder to bind the protein-containing food materials. All of these food products may be, for example, food products that can typically contain egg white.

Specific examples of the food products also include food products that are produced by using emulsifiers. Specific examples of food products produced by using emulsifiers include beverages such as dairy drinks, lactic acid bacteria beverages, carbonated drinks, vegetable drinks, fruit drinks, vegetable and fruit drinks, alcoholic drinks, coffee drinks, powdered drinks, sports drinks, supplement drinks, and tea drinks, ice confectionaries such as ice cream, soft ice cream, and ice candy, confectioneries such as puddings, jellies, bavarois, gums, chocolates, candy, cookies, biscuits, cakes, and donuts, breads, soups such as potage, liquid seasonings such as separate dressings, non-oil dressings, ketchup, dipping sauces, and sauces, processed meat products such as hams and sausages, processed seafood products such as fish sausages, hanpen, chikuwa, and kamaboko, dairy products such as yogurt, health foods and medical foods such as nutritional supplements, Foods with Nutrient Function Claims, Foods for Specified Health Uses, jams, preserves, syrups, fruits for processing, pickled vegetables, grain powders, noodles, liquid foods, etc. In these food products, the active ingredient may function, for example, as an emulsifier for emulsifying fats and oils, emulsifier for foaming food materials, and the like. All of these food products may be, for example, food products that can typically contain egg white.

The manner in which the food product is provided is not particularly limited. For example, the food product may be provided in a ready-to-consume form, or in a form that requires preparation before or at the time of eating, such as concentrated or dried products. The food product may also be provided in any container, such as retort pouches, paper cartons, plastic bottles such as PET bottles, metal cans such as steel or aluminum cans, and glass bottles. The food product is not limited to general food products, but also includes so-called health foods or medical foods, such as nutritional supplements, foods with nutrient function claims and foods for specified health uses. That is, for example, the food products exemplified above may be provided as general foods or as health foods or medical foods.

The term "raw materials of food products" means food materials for producing food products. The raw materials of food products is not particularly limited as long as it can be used to produce food products. The raw materials of food products can be selected according to various conditions, such as, for example, the type of food product. Examples of the raw materials of food products include raw materials that can be usually used in the production of food products such as those exemplified above. Specific examples of the raw materials of food products include grains such as rice and wheat flour; seasoning ingredients such as sugars, inorganic salts, organic acids, nucleic acids, amino acids, and protein hydrolysates; dairy products such as milk, cheese, and butter; protein-containing food materials such as meat and veggie meat; fruits; vegetables; eggs; spices; flavorings; fats and oils; alcohols; dietary fibers; and pH buffering agents. Examples of the eggs include eggs of birds such as chicken eggs. Examples of the eggs include any fraction of egg, such as whole egg, egg yolk, egg white, or a combination thereof. Examples of the eggs include a fraction containing at least egg yolk, e.g., whole egg or egg yolk. Examples of the eggs include a fraction containing at least egg white, e.g., whole egg or egg white. Eggs may be used in any form, such as liquid or powder.

The active ingredient may be added to the raw materials of food products at any stage of the food production process, as long as the desired effects, such as egg white substitution effect, binding effect, and emulsifying effect, can be obtained. That is, the "raw materials of food products" to which the active ingredient is added may be one at any stage of the food production process. For example, the "raw materials of food products" to which the active ingredient is added may be a finished food product before the active ingredient is added. The active ingredient can be added to the raw materials of food products as it is or in a desired form, such as a solution, as appropriate. The term "addition of the active ingredient" may be used to collectively refer to any operations to make the active ingredient coexist with the raw materials of food products. Ingredients other than the active ingredient may also be added to the raw materials of food products as appropriate. The descriptions concerning addition of the active ingredient may also be applied to addition of ingredients other than the active ingredient. All the respective ingredients (i.e., the active ingredient and optionally other ingredients) may be added to the raw materials of food products at the same time, or they may be added separately or separately in any combination thereof to the raw materials of food products. The order in which the respective ingredients are added to the raw materials of food products is not particularly limited.

The addition amounts and ratios of addition amounts of the ingredients (i.e., active ingredient and optionally other ingredients) used in the method of the present invention are not particularly limited as long as the desired effects such as egg white substitution effect, binding effect, and emulsifying effect can be obtained. The addition amounts and ratios of the addition amounts of the ingredients used in the method of the present invention can be appropriately set according to various conditions, such as the type of the raw materials of food products and the type of food product.

The active ingredient may be added to the raw materials of food products so that, for example, the content of the active ingredient in the food product is within a desired range (e.g., the range of content of the active ingredient as described below).

The content of the active ingredient in the food product may be, for example, 0.05% (w/w) or higher, 0.1% (w/w) or higher, 0.2% (w/w) or higher, 0.5% (w/w) or higher, 1% (w/w) or higher, 2% (w/w) or higher, 5% (w/w) or higher, 10% (w/w) or higher, 15% (w/w) or higher, or 20% (w/w) or higher, or may be 30% (w/w) or lower, 20% (w/w) or lower, 15% (w/w) or lower, 10% (w/w) or lower, 5% (w/w) or lower, 2% (w/w) or lower, 1% (w/w) or lower, 0.5% (w/w) or lower, 0.2% (w/w) or lower, or 0.1% (w/w) or lower, or may be any combination that does not contradict with the above-described ranges. The content of the active ingredient may specifically be, for example, 0.05 to 0.1% (w/w), 0.1 to 0.2% (w/w), 0.2 to 0.5% (w/w), 0.5 to 1% (w/w), 1 to 2% (w/w), 2 to 5% (w/w), 5 to 10% (w/w), 10 to 15% (w/w), 15 to 20% (w/w), or 20 to 30% (w/w). The content of the active ingredient may specifically be, for example, 0.05 to 30% (w/w), 0.1 to 20% (w/w), or 0.2 to 10% (w/w). When the food product of the present invention contains two or more types of serpins (e.g., when two or more types of serpins are added), each of the contents of those two or more types of serpins may be independently set or the total thereof may be set to be within any of the ranges of the content of the active ingredient exemplified above. When the food product of the present invention contains two or more types of serpins (e.g., when two or more types of serpins are added), the "content of the active ingredient" shall mean the total content of those two or more types of serpins, unless especially noted.

The active ingredient may be added to the raw materials of food products so that, for example, the concentration of the active ingredient at the time of eating is in a desired range (e.g., the range of the active ingredient concentration at the time of eating described below).

The concentration of the active ingredient at the time of eating may be, for example, 0.05% (w/w) or higher, 0.1% (w/w) or higher, 0.2% (w/w) or higher, 0.5% (w/w) or higher, 1% (w/w) or higher, 2% (w/w) or higher, 5% (w/w) or higher, 10% (w/w) or higher, 15% (w/w) or higher, or 20% (w/w) or higher, or may be 30% (w/w) or lower, 20% (w/w) or lower, 15% (w/w) or lower, 10% (w/w) or lower, 5% (w/w) or lower, 2% (w/w) or lower, 1% (w/w) or lower, 0.5% (w/w) or lower, 0.2% (w/w) or lower, or 0.1% (w/w) or lower, or may be any combination that does not contradict with the above-described ranges. The concentration of the active ingredient at the time of eating may be, specifically, for example, 0.05 to 0.1% (w/w), 0.1 to 0.2% (w/w), 0.2 to 0.5% (w/w), 0.5 to 1% (w/w), 1 to 2% (w/w), 2 to 5% (w/w), 5 to 10% (w/w), 10 to 15% (w/w), 15 to 20% (w/w), or 20 to 30% (w/w). The concentration of the active ingredient at the time of eating may be, specifically, for example, 0.05 to 30% (w/w), 0.1 to 20% (w/w), or 0.2 to 10% (w/w). When the food product of the present invention contains two or more types of serpins (e.g., when two or more types of serpins are added), the concentrations of these two or more kinds of serpins at the time of eating may be independently set or the total thereof may be set to be in any of the ranges of the concentration of the active ingredient at the time of eating exemplified above. When the food product of the present invention contains two or more types of serpins (e.g., when two or more types of serpins are added), the "concentration of the active ingredient at the time of eating" shall mean the total concentration of those two or more types of serpins at the time of eating, unless especially specified.

The descriptions concerning addition of the active ingredient can be applied to addition of the composition of the present invention. For example, the composition of the present invention can be added so that the addition amount of the active ingredient exemplified above can be obtained.

In one embodiment, the food product of the present invention may not contain egg white. That is, the food product of the present invention may be produced so as not to contain egg white.

In one embodiment, the food product of the present invention may contain egg white. That is, the food product of the present invention may be produced so as to contain egg white. Such a food product containing egg white may be produced by, for example, adding egg white. That is, the method of the present invention may further comprise adding egg white to a raw material of a food product. Egg white may be added in the same manner as the addition of the active ingredient. The food product containing egg white can be produced by, for example, using an egg white-containing raw material of a food product. That is, the raw materials of food products may contain egg white.

The food product of the present invention may be a food product typically containing egg white, but having a reduced egg white content. The term "food product having a reduced egg white content" means a food product having an egg white content lower than normal (i.e., food product having an egg white content lower than normal egg white content), and also means a food product containing no egg white at all. A food product containing a normal amount of egg white (i.e., food product having a normal egg white content) is also referred to as "normal egg white-containing food product". That is, the "food product having a reduced egg white content" may specifically mean a food product of the same type as a normal egg white-containing food product, but having an egg white content lower than that of the normal egg white-containing food product. The term "food product having a reduced egg white content" may mean, more specifically, a food product of the same type as a normal egg white-containing food product having an egg white content 0.9 times or less, 0.8 times or less, 0.7 times or less, 0.6 times or less, 0.5 times or less, 0.4 times or less, 0.3 times or less, 0.2 times or less, or 0.1 times or less of the egg white content of the normal egg white-containing food product, and may also mean a food product that does not contain egg white at all. Examples of such food products having reduced egg white contents include food products typically containing egg white such as the food products exemplified above, which are produced so as to have an egg white content lower than normal or so as not to contain egg white. Examples of normal egg white-containing food products include egg white-containing food products such as the food products exemplified above, which are produced so as to contain a normal amount of egg white. According to the present invention, for example, a decrease in the function of egg white due to a reduction in egg white content may be compensated. That is, "substitution of egg white" may include compensation of reduced function of egg white due to a reduced content of egg white.

<4> Use of the active ingredient

The present invention also relates to use of the active ingredient in the applications exemplified above. That is, the present invention relates to, for example, use of the active ingredient for substituting egg white, binding a raw material of a food product, emulsifying fats and oils in a raw material of a food product, or producing a food product, or use of the active ingredient in production of a composition for substituting egg white, binding a raw material of a food product, emulsifying fats and oils in a raw material of a food product, or producing a food product.

The present invention also relates to the active ingredient for use in the applications exemplified above. That is, the present invention relates to, for example, the active ingredient for use in substitution of egg white, binding of a raw material of a food product, emulsification of fats and oils in a raw material of a food product, or production of a food product, or the active ingredient for use in production of a composition for substituting egg white, binding a raw material of a food product, emulsifying fats and oils in a raw material of a food product, or producing a food product.

EXAMPLES

Hereafter, the present invention will be explained more specifically with reference to the following non-limiting examples.

(Example 1) Preparation of serpin and measurement of elasticity

(1-1) Expression and purification of serpin

As serpin, there were selected serpin derived from *Arabidopsis thaliana* (AtSerpin), serpin derived from *Brassica napus* (BnSerpin), serpin derived from *Theobroma cacao* (TcSerpin), serpin derived from *Ricinus communis* (RcSerpin), serpin derived from *Helianthus annuus* (HaSerpin), serpin derived from *Camellia sinensis* var. *sinensis* (CsSerpin), and serpin derived from *Daucus carota* subsp. *sativus* (DcSerpin).

The method for preparing serpin derived from *Arabidopsis thaliana* (Ath) (AtSerpin) will be shown below.

DNA containing the nucleotide sequence encoding AtSerpin was obtained by total synthesis and inserted into pET-21b at the NdeI-HindIII restriction enzyme site to construct an AtSerpin expression plasmid pET-AtSerpin (construction was subcontracted to Eurofins Genomics K. K.). The nucleotide sequence encoding AtSerpin and the amino acid sequence of AtSerpin are shown in SEQ ID NO: 1 and SEQ ID NO: 2, respectively. The nucleotide sequence of pET-21b(+) is shown as the sequence of SEQ ID NO: 19. AtSerpin expressed from pET-AtSerpin has a histidine tag derived from pET-21b(+).

The AtSerpin expression plasmid pET-AtSerpin was introduced into the *E. coli* BL21 (DE3) strain (Sigma-Aldrich) to obtain BL21 (DE3)/pET-AtSerpin strain. Transformation using heat shock was performed as described in the protocol. This strain was inoculated into the LB medium (1.0% (w/v) peptone, 0.50% (w/v) yeast extract, and 1.0% (w/v) NaCl) supplemented with 100 $\mu$g/mL ampicillin and cultured overnight at 37°C with shaking. Subsequently, the culture was inoculated into 100 mL of the LB medium contained in a Sakaguchi flask at a final concentration of 1%. After culture with shaking at 37°C for 3 hours, isopropyl-$\beta$-D-thiogalactopyranoside (IPTG) was added at a final concentration of 1.0 mM, and the culture was continued for another 2 hours.

The whole culture was centrifuged at 9,100 x g for 5 minutes to collect the bacterial cells, and the cells were washed twice with 10 mL of 10 mM Tris-HCl (pH 8.0) buffer, and suspended in 50 mL of 1% NaCl aqueous solution. The resulting suspension was subjected to a cell disruption treatment (170 W, 4°C, 10 minutes) in an ultrasonicator (Kubota Shoji), and centrifuged at 9,100 x g for 10 minutes to obtain a pellet fraction.

The obtained pellet fraction consisted of inclusion bodies containing AtSerpin insolubilized in *E. coli* cells. A solubilizing solution (50 mL) of 10 mM Tris-HCl (pH 8.0), 8 M urea, 5 mM DTT was added to the fraction, and the mixture was gently stirred at room temperature for 1 hour to obtain a solubilized solution.

The resulting solubilized solution (45 mL) was adsorbed on a column (Ni Sepharose Fast Flow 16/10, 20 mL, Cytiva) equilibrated with 10 mM Tris (pH 8.0), 160 mM urea, and 5.0 mM imidazole. Washing was then performed with the equilibration buffer in 9 column volumes. Further, the equilibration buffer containing 500 mM imidazole and 8 M urea (elution buffer) was passed through the column, and the fraction containing AtSerpin was collected, yielding 36 mL of purified AtSerpin at about 1.0 mg/mL.

The obtained purified AtSerpin was desalted using Sephadex G-25, HiScale 26/20 (324 mL). Sephadex G-25 was equilibrated in advance with an equilibration solution, 1 mM Tris-HCl (pH 8.0), in a volume three times the volume of column resin, and after the purified AtSerpin was added, the equilibration solution in a volume 5 times the volume of column resin was passed through the column. A fraction containing AtSerpin was fractionated to obtain 100 mL of an about 0.3 mg/mL fraction as desalted AtSerpin.

The resulting desalted AtSerpin was concentrated by using a UF cartridge: Amicon Ultra 15, 10 kDa (Millipore) to obtain approximately 10 mL of concentrated AtSerpin.

The solvent was removed from the obtained concentrated AtSerpin in a lyophilizer Genesis 25EL (SP Industries) to obtain dried AtSerpin.

In the same manner, expression plasmids for BnSerpin, TcSerpin, RcSerpin, HaSerpin, DcSerpin, and CsSerpin were constructed, and heterologous expression thereof was carried out in *E. coli* to obtain dried BnSerpin, dried TcSerpin, dried RcSerpin, dried HaSerpin, dried CsSerpin, and dried DcSerpin. The nucleotide sequence encoding BnSerpin and the amino acid sequence of BnSerpin are shown as the sequences of SEQ ID NOS: 3 and 4, respectively. The nucleotide sequence encoding TcSerpin and the amino acid sequence of TcSerpin are shown as the sequences of SEQ ID NOS: 5 and 6, respectively. The nucleotide sequence encoding RcSerpin and the amino acid sequence of RcSerpin are shown as the sequences of SEQ ID NOS: 7 and 8, respectively. The nucleotide sequence encoding HaSerpin and the amino acid sequence of HaSerpin are shown as the sequences of SEQ ID NOS: 13 and 14, respectively. The nucleotide sequence encoding CsSerpin and the amino acid sequence of CsSerpin are shown as the sequences of SEQ ID NOS: 15 and 16, respectively. The nucleotide sequence encoding DcSerpin and the amino acid sequence of DcSerpin are shown as the sequences of SEQ ID NOS: 17 and 18, respectively.

(1-2) Measurement of elasticity of serpin during heating

The elasticity of serpins obtained in Example 1 during heating was measured.

Each dried serpin was dissolved in water at a concentration of 12% (wt/v) or 15% (wt/v) to prepare a serpin solution. The solution was mounted on the measurement table of a rheometer, Discovery HR20 (TA Instrument), at the center position directly below the parallel plate, 40 mm SMART-SWAP HRx0 (TA Instruments), and the parallel plate positioned above was vertically lowered toward the sample to make contact, and then the sample was heated from 25°C to 100°C (temperature increase rate 5°C/minute) at a strain of 1% and a frequency of 1 Hz, and storage modulus analysis was performed under the above measurement conditions.

The results are shown in Table 1. All the serpins gelled and exhibited elasticity.

[Table 1]

Table 1

| Origin of Serpin | Sample code | Gelation by heating | Rheology analysis Storage elasticity (Pa) |
|---|---|---|---|
| *Arabidopsis thaliana* | AtSerpin | Gelled | 812 |
| *Brassica napus* | BnSerpin | Gelled | 722 |
| *Theobroma cacao* | TcSerpin | Gelled | 812 |
| *Ricinus communis* | RcSerpin | Gelled | 1184 |
| *Helianthus annuus* | HaSerpin | Gelled | 590 |
| *Camellia sinensis* var. *sinensis* | CsSerpin | Gelled | 1085 |
| *Daucus carota* subsp. *sativus* | DcSerpin | Gelled | 763 |

(Example 2) Preparation of pâté-like food products and measurements of breaking strength thereof and emulsifying power

(2-1) Preparation of pâté-like food product

The methods for preparing pâté-like food products without additives, pâté-like food products containing egg white, and pâté-like food products containing serpin will be shown below.

A rehydration solution was prepared by mixing vegetable oil and water. Then, New Fujinic® 80N and Apex® 350 (both produced by Fuji Oil Co., Ltd.) were rehydrated with the rehydration solution, and New Fujinic® 80N and Apex® 350 were mixed at a ratio of 9:4 to prepare granular soy protein. Apex® 950 (Fuji Oil Co., Ltd.) was also rehydrated with the rehydration solution and then crushed with a blender until it became flakes to prepare fibrous soy protein.

The granular soy protein and fibrous soy protein were mixed to prepare a base protein, then the base protein was mixed with methylcellulose powder (pâté-like food without additives), methylcellulose powder and egg white powder (pâté-like food containing egg white), or methylcellulose powder and each dried serpin obtained in Example 1 (pâté-like food containing serpin) as the binder, and further mixed with fats and oils and seasonings to prepare various pâtélike food products. Each pâté-like food product was molded, baked at 180°C, then flash-frozen, and stored frozen in a vacuum pouch. The raw materials of the pâté-like food products and formulation ratios thereof are shown in Table 2.

[Table 2]

Table 2

| Raw material | | Formulation ratio (%) | | |
|---|---|---|---|---|
| | | No additive | Containing egg white | Containing serpin |
| Binder | Methylcellulose powder | 0.3 | 0.3 | 0.3 |
| | Egg white powder | N/A | 1.0 | N/A |
| | Dried serpin | N/A | N/A | 1.0 |
| Base protein | Granular protein | 13.0 | 13.0 | 13.0 |
| | Fibrous protein | 9.5 | 9.5 | 9.5 |
| Rehydration solution | Water | 55.4 | 55.4 | 55.4 |
| | Vegetable oil | 9.5 | 9.5 | 9.5 |
| Fat or oil | Solid vegetable fats and oils | 10.0 | 10.0 | 10.0 |

(continued)

| Raw material | | Formulation ratio (%) | | |
|---|---|---|---|---|
| | | No additive | Containing egg white | Containing serpin |
| Seasoning | Salt | 1.0 | 1.0 | 1.0 |
| | Onion powder | 0.3 | 0.3 | 0.3 |
| | Garlic powder | 0.3 | 0.3 | 0.3 |
| | Black pepper | 0.2 | 0.2 | 0.2 |
| | Nutmeg | 0.3 | 0.3 | 0.3 |
| | Sugar | 0.2 | 0.2 | 0.2 |
| | Total | 100.0 | 100.0 | 100.0 |

(2-2) Measurement of breaking strength of pâté-like food product

Breaking strength of each pâté-like food product obtained in (2-1) was measured by using a texture analyzer (TA, TA-XT-plus, Stable Micro Systems). Specifically, stress (g) required at the time of breaking horizontally placed each pâté-like food product with a wedge-shaped plunger was measured.

The measured breaking strengths of the pâté-like food products are shown in Table 3. The serpin-containing pâté-like food products showed similar breaking strength to that of the egg white-containing pâté-like food product, regardless of which serpin was used.

[Table 3]

Table 3

| Sample | Breaking strength (g) |
|---|---|
| Pâté-like food product without additives | 343 |
| Pâté-like food product containing egg white | 899 |
| Pâté-like food product containing AtSerpin | 812 |
| Pâté-like food product containing HaSerpin | 626 |
| Pâté-like food product containing DcSerpin | 582 |

(2-3) Measurement of emulsifying effect of each serpin

In 100 g of water, 5 g of each dried serpin obtained in Example 1, casein (Junsei Chemical), bovine serum albumin (Sigma-Aldrich), or egg white albumin (Sigma-Aldrich) were dissolved as an emulsifier. Then, 100 g of canola oil (J-Oil Mills) was added to the solution, and the mixture was stirred with a mixer to prepare an emulsion. The prepared emulsion was slowly transferred into a 500 mL graduated cylinder, and allowed to stand at room temperature for 1 hour. After 1 hour, the height of the entire emulsion (HE) and the height of the transparent layer (HS) were measured. The emulsion was then allowed to stand at 4°C for 24 hours, and the HE and HS were measured again. From the measured values of HE and HS, the creaming index (CI) was calculated in accordance with the following equation.

$$\text{Creaming Index (CI)} = \text{HS/HE}$$

The calculated values of the creaming index (CI) for the samples are shown in Table 4. Each serpin gave creaming indexes (CI) comparable to or higher than that obtained with casein.

[Table 4]

Table 4

| Emulsifier | Creaming index (%) | |
| --- | --- | --- |
| | After 1 hour | After 24 hours |
| Casein | 47 | 47 |
| Bovine serum albumin | 36 | 72 |
| Egg white albumin | 56 | 96 |
| AtSerpin | 9 | 42 |
| HaSerpin | 43 | 45 |
| DcSerpin | 45 | 47 |

<Sequence Listing>

SEQ ID NO: 1, Nucleotide sequence encoding serpin derived from *Arabidopsis thaliana* (AtSerpin)
SEQ ID NO: 2, Amino acid sequence of serpin derived from *Arabidopsis thaliana* (AtSerpin)
SEQ ID NO: 3, Nucleotide sequence encoding serpin derived from *Brassica napus* (BnSerpin)
SEQ ID NO: 4, Amino acid sequence of serpin derived from *Brassica napus* (BnSerpin)
SEQ ID NO: 5, Nucleotide sequence encoding serpin derived from *Theobroma cacao* (TcSerpin)
SEQ ID NO: 6, Amino acid sequence of serpin derived from *Theobroma cacao* (TcSerpin)
SEQ ID NO: 7, Nucleotide sequence encoding serpin derived from *Ricinus communis* (RcSerpin)
SEQ ID NO: 8, Amino acid sequence of serpin derived from *Ricinus communis* (RcSerpin)
SEQ ID NO: 9, Nucleotide sequence encoding serpin derived from *Brassica rapa* subsp. *pekinensis* (BrSerpin)
SEQ ID NO: 10, Amino acid sequence of serpin derived from *Brassica rapa* subsp. *pekinensis* (BrSerpin)
SEQ ID NO: 11, Nucleotide sequence encoding serpin derived from *Raphanus sativus* (RsSerpin)
SEQ ID NO: 12, Amino acid sequence of serpin derived from *Raphanus sativus* (RsSerpin)
SEQ ID NO: 13, Nucleotide sequence encoding serpin derived from *Helianthus annuus* (HaSerpin)
SEQ ID NO: 14, Amino acid sequence of serpin derived from *Helianthus annuus* (HaSerpin)
SEQ ID NO: 15, Nucleotide sequence encoding serpin derived from *Camellia sinensis* var. *sinensis* (CsSerpin)
SEQ ID NO: 16, Amino acid sequence of serpin derived from *Camellia sinensis* var. *sinensis* (CsSerpin)
SEQ ID NO: 17, Nucleotide sequence encoding serpin derived from *Daucus carota* subsp. *sativus* (DcSerpin)
SEQ ID NO: 18, Amino acid sequence of serpin derived from *Daucus carota* subsp. *sativus* (DcSerpin)
SEQ ID NO: 19, Nucleotide sequence of pET-21b (+)

**Claims**

1.  A composition comprising a serpin derived from a plant or fungus, for substituting egg white, for binding a food material, for emulsifying fats and oils in a food material, or for producing a food product.

2.  The composition according to claim 1, wherein the serpin is a serpin derived from a plant of the family *Brassicaceae, Malvaceae, Euphorbiaceae, Asteraceae, Theaceae,* or *Apiaceae.*

3.  The composition according to claim 1 or 2, wherein the serpin is a serpin derived from a plant of the genus *Arabidopsis, Brassica, Theobroma, Ricinus, Helianthus, Camellia,* or *Daucus.*

4.  The composition according to claim 1 or 2, wherein the serpin is a serpin derived from *Arabidopsis thaliana, Brassica napus, Theobroma cacao, Ricinus communis, Helianthus annuus, Camellia sinensis* var. *sinensis,* or *Daucus carota* subsp. *sativus.*

5. The composition according to claim 1 or 2, wherein the serpin is the following protein (a), (b), or (c):

(a) a protein comprising the amino acid sequence of SEQ ID NO: 2, 4, 6, 8, 14, 16, or 18;
(b) a protein comprising an amino acid sequence of SEQ ID NO: 2, 4, 6, 8, 14, 16, or 18, but which includes substitution, deletion, insertion, and/or addition of 1 to 10 amino acid residues, wherein said protein has an egg white substitution function, a binding function, or an emulsifying function; or
(c) a protein comprising an amino acid sequence having an identity of 90% or higher to the amino acid sequence of SEQ ID NO: 2, 4, 6, 8, 14, 16, or 18, wherein said protein has an egg white substitution function, a binding function, or an emulsifying function.

6. The composition according to claim 1 or 2, wherein the serpin has a molecular weight of 40,000 to 50,000.

7. The composition according to claim 1 or 2, wherein the serpin has one or more properties selected from the following properties (1) to (4):

(1) the ratio of Ser residues in the amino acid sequence is 7.5% or higher;
(2) the isoelectric point (pI) is 4 to 7;
(3) the interior hydrophobicity is 1.5 or higher; and
(4) the predicted scaled solubility is 0.45 or higher.

8. A method for producing a food product, the method comprising:
adding a serpin derived from a plant or fungus to a raw material of a food product.

9. The method according to claim 8, wherein the food product to be produced is a food product in which egg white has been substituted, a food material has been bound, and/or fats and oils in a food material have been emulsified.

10. A method for substituting egg white in a food product, the method comprising:
adding a serpin derived from a plant or fungus to a raw material of a food product.

11. A method for binding a food material, the method comprising:
adding a serpin derived from a plant or fungus to a raw material of a food product.

12. A method for emulsifying fats and oils in a food material, the method comprising:
adding a serpin derived from a plant or fungus to a raw material of a food product.

13. The method according to any one of claims 8 to 12, wherein the serpin is a serpin derived from a plant of the family *Brassicaceae, Malvaceae, Euphorbiaceae, Asteraceae, Theaceae,* or *Apiaceae.*

14. The method according to any one of claims 8 to 12, wherein the serpin is a serpin derived from a plant of the genus *Arabidopsis, Brassica, Theobroma, Ricinus, Helianthus, Camellia,* or *Daucus.*

15. The method according to any one of claims 8 to 12, wherein the serpin is a serpin derived from *Arabidopsis thaliana, Brassica napus, Theobroma cacao, Ricinus communis, Helianthus annuus, Camellia sinensis* var. *sinensis,* or *Daucus carota* subsp. *sativus.*

16. The method according to any one of claims 8 to 12, wherein the serpin is the following protein (a), (b), or (c):

(a) a protein comprising the amino acid sequence of SEQ ID NO: 2, 4, 6, 8, 14, 16, or 18;
(b) a protein comprising an amino acid sequence of SEQ ID NO: 2, 4, 6, 8, 14, 16, or 18, but which includes substitution, deletion, insertion, and/or addition of 1 to 10 amino acid residues, wherein said protein has an egg white substitution function, a binding function, or an emulsifying function; or
(c) a protein comprising an amino acid sequence having an identity of 90% or higher to the amino acid sequence of SEQ ID NO: 2, 4, 6, 8, 14, 16, or 18, wherein said protein has an egg white substitution function, a binding function, or an emulsifying function.

17. The method according to any one of claims 8 to 12, wherein the serpin has a molecular weight of 40,000 to 50,000.

18. The method according to any one of claims 8 to 12, wherein the serpin has one or more properties selected from the following properties (1) to (4):

> (1) the ratio of Ser residues in the amino acid sequence is 7.5% or higher;
> (2) the isoelectric point (pI) is 4 to 7;
> (3) the interior hydrophobicity is 1.5 or higher; and
> (4) the predicted scaled solubility is 0.45 or higher.

19. The method according to any one of claims 8 to 12, wherein the serpin is added so that the concentration thereof at the time of eating is 0.05 to 30% (w/w).

20. A food product comprising a serpin derived from a plant or fungus.

21. The food product according to claim 20, wherein the serpin is a serpin derived from a plant of the family *Brassicaceae, Malvaceae, Euphorbiaceae, Asteraceae, Theaceae,* or *Apiaceae.*

22. The food product according to claim 20 or 21, wherein the serpin is a serpin derived from a plant of the genus *Arabidopsis, Brassica, Theobroma, Ricinus, Helianthus, Camellia,* or *Daucus.*

23. The food product according to claim 20 or 21, wherein the serpin is a serpin derived from *Arabidopsis thaliana, Brassica napus, Theobroma cacao, Ricinus communis, Helianthus annuus, Camellia sinensis var. sinensis,* or *Daucus carota* subsp. *sativus.*

24. The food product according to claim 20 or 21, wherein the serpin is the following protein (a), (b), or (c):

> (a) a protein comprising the amino acid sequence of SEQ ID NO: 2, 4, 6, 8, 14, 16, or 18;
> (b) a protein comprising an amino acid sequence of SEQ ID NO: 2, 4, 6, 8, 14, 16, or 18, but which includes substitution, deletion, insertion, and/or addition of 1 to 10 amino acid residues, wherein said protein has an egg white substitution function, a binding function, or an emulsifying function; or
> (c) a protein comprising an amino acid sequence having an identity of 90% or higher to the amino acid sequence of SEQ ID NO: 2, 4, 6, 8, 14, 16, or 18, wherein said protein has an egg white substitution function, a binding function, or an emulsifying function.

25. The food product according to claim 20 or 21, wherein the serpin has a molecular weight of 40,000 to 50,000.

26. The food product according to claim 20 or 21, wherein the serpin has one or more properties selected from the following properties (1) to (4):

> (1) the ratio of Ser residues in the amino acid sequence is 7.5% or higher;
> (2) the isoelectric point (pI) is 4 to 7;
> (3) the interior hydrophobicity is 1.5 or higher; and
> (4) the predicted scaled solubility is 0.45 or higher.

27. The food product according to claim 20 or 21, wherein the food product has a serpin content of 0.05 to 30% (w/w).

## INTERNATIONAL SEARCH REPORT

| International application No. |
|---|
| **PCT/JP2024/023348** |

**A. CLASSIFICATION OF SUBJECT MATTER**

*A23J 3/14*(2006.01)i; *A23J 3/00*(2006.01)i; *A23L 29/10*(2016.01)i; *A23L 33/185*(2016.01)i; *A23L 33/195*(2016.01)i; *C07K 14/37*(2006.01)i; *C07K 14/81*(2006.01)i; *C07K 14/415*(2006.01)i; *C12N 15/29*(2006.01)i

FI: A23J3/14; A23J3/00 501; A23J3/00 508; A23L29/10; A23L33/185; A23L33/195; C07K14/37 ZNA; C07K14/415; C07K14/81; C12N15/29

According to International Patent Classification (IPC) or to both national classification and IPC

**B. FIELDS SEARCHED**

Minimum documentation searched (classification system followed by classification symbols)

A23J3/14; A23J3/00; A23L29/10; A23L33/185; A23L33/195; C07K14/37; C07K14/81; C07K14/415; C12N15/29

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 11-147834 A (NOEVIR CO., LTD.) 02 June 1999 (1999-06-02) particularly, claims 1-5, paragraph [0016], examples | 1, 8, 9, 20 |
| Y | particularly, claims 1-5, paragraph [0016], examples | 1-9, 11, 13-27 |
| A | particularly, claims 1-5, paragraph [0016], examples | 10, 12 |
| Y | ROBERTS T.H. and HEJGAARD J., Serpins in plants and green algae, Funct Integr Genomics, 2008, vol. 8, pp. 1-27 particularly, abstract, page 1, right column, first paragraph - page 2, right column, fourth paragraph, table 1 | 1-9, 11, 13-27 |
| A | particularly, abstract, page 1, right column, first paragraph - page 2, right column, fourth paragraph, table 1 | 10, 12 |

☑ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **14 August 2024** | **27 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/023348** |

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| Y | SINGH A. and BENJAKUL S., Serine protease inhibitors from squid ovary: extraction and its effect on proteolysis and gel properties of surimi, J Food Sci Technol, 2017, vol. 54, pp. 267-275<br>    particularly, abstract, page 267, right column, first paragraph | 1-9, 11, 13-27 |
| A |     particularly, abstract, page 267, right column, first paragraph | 10, 12 |
| A | JP 2011-79768 A (NATIONAL INSTITUTE OF ADVANCED INDUSTRIAL SCIENCE AND TECHNOLOGY) 21 April 2011 (2011-04-21)<br>    entire text | 1-27 |

**INTERNATIONAL SEARCH REPORT**

International application No.

**PCT/JP2024/023348**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

| International application No. |
|---|
| **PCT/JP2024/023348** |

| Patent document cited in search report | | | Publication date (day/month/year) | Patent family member(s) | Publication date (day/month/year) |
|---|---|---|---|---|---|
| JP | 11-147834 | A | 02 June 1999 | (Family: none) | |
| JP | 2011-79768 | A | 21 April 2011 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NARDY LAMPL et al.** Arabidopsis AtSerpin1, Crystal Structure and in Vivo Interaction with Its Target Protease RESPONSIVE TO DESICCATION-21 (RD21). *J Biol Chem.*, 30 April 2010, vol. 285 (18), 13550-13560

- **NIWA TATSUYA et al.** Bimodal protein solubility distribution revealed by an aggregation analysis of the entire ensemble of Escherichia coli proteins. *Proc Natl Acad Sci USA*, vol. 106, 4201-4206
- **KYTE, J. ; DOOLITTLE, R. F.** A simple method for displaying the hydropathic character of a protein.. *J Mol Biol*, 1982, vol. 157 (1), 105-132